# EUROPÄISCHE PATENTSCHRIFT

(11) **EP 3 393 624 B1**
(45) Veröffentlichungstag und Bekanntmachung des Hinweises auf die Patenterteilung: **22.05.2019**
(21) Anmeldenummer: 15750926.6
(22) Anmeldetag: 28.07.2015
(51) Int. Cl.: B01D 53/14, C12M 1/00, B01D 19/00, B01J 19/12

(54) **VERFAHREN UND VORRICHTUNG ZUR SELEKTIVEN ENTGASUNG VON METHAN AUS WASCHFLÜSSIGKEIT**
METHOD AND DEVICE FOR SELECTIVELY DEGASSING METHANE FROM SCRUBBING LIQUID
PROCÉDÉ ET DISPOSITIF SERVANT AU DÉGAZAGE SÉLECTIF DU MÉTHANE D'UN LIQUIDE DE LAVAGE

(30) Priorität: 08.08.2014 DE 102014011529
(43) Veröffentlichungstag der Anmeldung: 31.10.2018
(73) Patentinhaber: Klein, Harald, 37217 Witzenhausen (DE)
(72) Erfinder: Klein, Harald, 37217 Witzenhausen (DE)
(74) Vertreter: Lindinger, Bernhard
(86) Internationale Anmeldenummer: PCT/EP2015/001552
(87) Internationale Veröffentlichungsnummer: WO 2016/020045

(56) Entgegenhaltungen:
- EP-A1- 1 504 813
- EP-A1- 2 570 164
- EP-A1- 2 609 988
- WO-A1-2012/030368
- DE-A1-102011 102 923
- DE-U1-202014 007 616

## Beschreibung

### Gebiet der Technik:

Die Erfindung bezieht sich auf ein Verfahren und eine Vorrichtung und deren Verwendung zur Entgasung von Methan aus einer auch Kohlendioxid enthaltenden überwiegend aus Wasser bestehenden Waschflüssigkeit, wie insbesondere bei einer Druckwasserwäsche, sowie eine entsprechende Verwendung der Vorrichtung. Druckwasserwaschverfahren werden vor allem in Biogasanlagen zur Reinigung des Rohbiogases eingesetzt. Bei einer Druckwasserwäsche wird vor allem Kohlendioxid aus einem Gasgemisch, wie beispielsweise circa 52% Methan und circa 48% Kohlenstoff enthaltendes Rohbiogas, mit Druck beaufschlagter Waschflüssigkeit, die vorwiegend aus Wasser besteht, ausgewaschen. Normalerweise wird das Gasgemisch im Gegenstromprinzip mit der Waschflüssigkeit gewaschen. Aus DE102005051952B3 ist ein Verfahren zur Herstellung von Methan und flüssigem Kohlendioxid aus Raffineriegas und/oder Biogas bekannt. Mit dem Druckwasserwaschverfahren lässt sich Rohbiogas zu Biomethan mit circa 98% Methangehalt aufbereiten. Bei dem Waschprozess wird jedoch auch ein geringer Anteil Methan von ungefähr 0,5 % bis ungefähr
2 % des ausgewaschenen Gases in der Waschflüssigkeit mitgerissen und in der Desorption, gemeinsam mit dem Kohlendioxid, als Abgas in die Atmosphäre entlassen. Dieses mit Methan verunreinigte Abgas stellt sowohl ein umwelttechnisches als auch ein emissionsrechtliches Problem dar.

Eine beispielsweise nach dem Absorptionsschritt eines Druckwasserwäscheverfahrens Methan und Kohlendioxid enthaltende überwiegend aus Wasser bestehende Waschflüssigkeit ist eine Waschflüssigkeit, die zumindest über die Hälfte ihrer Mengenanteile aus Wasser besteht. So kann die Waschflüssigkeit bis auf das enthaltende Methan und Kohlendioxid nur aus Wasser bestehen oder aber auch mit Zusätzen versetzt sein, soweit dabei der Anteil des Wassers in der Waschflüssigkeit zumindest noch über der Hälfte liegt. Zusätze zum Wasser sind für Waschflüssigkeiten bekannt, wie zum Beispiel Diethanolamin. Die Größe der Anteile von Zusätzen in der Waschflüssigkeit kann also je nach Art der Waschflüssigkeit im Bereich von Null bis zur Grenze, bei der noch gerade der Anteil des Wassers über die Hälfte ausmacht, liegen. Beispielsweise wird bei dem in WO2008/092604 offenbarten Verfahren Waschflüssigkeit mit einer Diethanolaminkonzentation von 15 % bis 50 % verwendet.

### Stand der Technik:

Für in großer Tiefe bei hohem statischen Druck im Wasser enthaltenes Methan sind Verfahren zur Entgasung des Methans aus dem Wasser bekannt, bei denen mittels verringertem statischen Druck, der auf das Wasser einwirkt, das Methan entgast wird. So wird in WO2004/103913A1 ein Verfahren und eine Vorrichtung offenbart, bei denen die Bildung von Gasblasen im gelöstes Methan enthaltendem Wasser durch Ultraschall angeregt wird. Auch sind Methoden zur Entgasung von Waschflüssigkeit bei Druckwasserwäschen durch Druckveränderungen wie beispielsweise bei in EP2570164A1 offenbartem Einsatz von Zentrifugalabscheidern bekannt. Auch bei dem in WO2008/092604A1 offenbarten Verfahren wird die Entgasung der Waschflüssigkeit mittels Druckveränderungen durchgeführt. Ferner ist ein Verfahren und eine Vorrichtung einer Ultraschallbehandlung von Waschflüssigkeit zur Entgasung von Kohlendioxid aus Waschflüssigkeit in DE102008039171A1 offenbart. Jedoch wird dort nicht zwischen verschiedenen in der Waschflüssigkeit gelösten Gasen unterschieden. In US2009/0156875A1 wird zwar eine Abtrennung von Methan aus einer kohlendioxidhaltigen Waschflüssigkeit erwähnt, jedoch keine näheren Angaben, auf welche Art und Weise dieses erfolgt, gemacht. Nachteil bei bisherigen Reinigungs- oder Desorptionsverfahren von Waschflüssigkeit bei einer Druckwasserwäsche und Vorrichtungen dazu ist vor allem der zu hohe Anteil von Methan im vorwiegend Kohlendioxid enthaltenden Abgas. Der im Abgas prozentual zu hohe Methananteil muss dann aus umwelttechnischen und emissionsrechtlichen Gründen durch kosten- und energieintensive chemische, thermische und biologische Behandlung des Abgases reduziert werden. Es ist bisher noch kein effektives Verfahren und keine Vorrichtung zur selektiven Reduzierung des in der Waschflüssigkeit einer Druckwasserwäsche gelösten Methananteils im Verhältnis zum gelösten Kohlendioxidanteil bekannt. Sehr allgemein wird in WO2012/030368A1 ein Verfahren und eine Vorrichtung zur Trennung von Komponenten in einer Flüssigkeit durch Einwirken von elektromagnetischen Wellen im Radiofrequenzbereich auf die Flüssigkeit offenbart, wobei die Frequenz ungefähr der Vibrationsfrequenz des von der zu trennenden Komponente der Flüssigkeit entspricht. In WO2012/030368A1 wird jedoch weder speziell eine Verfahren oder Vorrichtung zum Entgasung von Methan aus einer Waschflüssigkeit erwähnt noch eine Lösung für eine Abgasproblematik bei einer Druckwasserwäsche vorgeschlagen.

In EP 2 609 988 A1 ist ein Niedrigtemperaturverfahren zur Desorption aminhaltiger Waschflüssigkeiten aus Gaswaschprozessen sowie eine Vorrichtung dazu offenbart. Bei dem dortigen Niedrigtemperaturverfahren mit der Vorrichtung wird die Waschflüssigkeit verdichtet, erwärmt, zur weiteren Bildung von Nukleationskeimen angeregt, dann wieder entspannt und in eine Verweil- und Absetzzone zum Entgasen gebracht sowie danach wieder zumindest zu einem Teil der Verdichtungszone im Umlauf zugeführt. Eine selektive Entgasung von Methan aus der Waschflüssigkeit ist dort nicht erwähnt. Hinsichtlich elektromagnetischer Wellen wird in EP 2 609 988 A1 lediglich eine elektromagnetische Erwärmung im RF- bzw. Mikrowellenbereich zur Schaumminderung angeführt.

In EP 1 504 813 A1 ist ein Verfahren zur Plasmabildung in einer Flüssigkeit sowie eine Vorrichtung dazu offenbart. Dabei werden mit einem Ultraschallgenerator Blasen in der Flüssigkeit erzeugt bzw. zur Schwingung angeregt und die Flüssigkeit samt Blasen mit elektromagnetischen Wellen bestrahlt. Die dortige Vorrichtung ist weder zur Entgasung von Methan aus Waschflüssigkeit geeignet noch vorgesehen, sondern für Zersetzung von giftigen Substanzen. Es wird mit der Vorrichtung in EP 1 504 813 A1 ein Plasma in der Flüssigkeit gebildet. Die Frequenzen der elektromagnetischen Wellen liegen in EP 1 504 813 A1 hauptsächlich bei 2 GHz und höher.

EP 2 570 164 A1 offenbart eine Vorrichtung und ein Verfahren zur Entgasung von Methan aus einer auch Kohlendioxid enthaltenden, überwiegend aus Wasser bestehenden Waschflüssigkeit, wobei durch gewisse mechanische/thermische Behandlung die Bindung des Methans in der Waschflüssigkeit verringerbar und/oder das Methan in der Waschflüssigkeit zur Bewegung anregbar ist.

Der Erfindung liegt daher die Aufgabe zugrunde, ein verbessertes Verfahren und entsprechend eine verbesserte Vorrichtung zur Entgasung von Methan aus einer auch Kohlendioxid enthaltenden überwiegend aus Wasser bestehenden Waschflüssigkeit bereitzustellen sowie eine solche Verwendung einer solchen Vorrichtung bereitzustellen.

### Zusammenfassung der Erfindung:

Die Aufgabe wird durch ein Verfahren gemäß den Merkmalen des Anspruch 1 sowie entsprechend durch eine Vorrichtung gemäß den Merkmalen des Anspruchs 11 sowie entsprechend durch eine Verwendung der Vorrichtung gemäß den Merkmalen des Anspruchs 15 gelöst.

Das Verfahren zur Entgasung von Methan aus einer auch Kohlendioxid enthaltenden überwiegend aus Wasser bestehenden Waschflüssigkeit, wie insbesondere bei einer Druckwasserwäsche, ist durch das Einwirken von elektromagnetischen Wellen beziehungsweise von elektromagnetischem Wechselfeld auf die Waschflüssigkeit mit zumindest einer Frequenz innerhalb eines Frequenzbereichs von 0,5 bis 10 Megahertz, wobei bei der zumindest einen Frequenz durch das Einwirken die Bindung des Methans in der Waschflüssigkeit verringerbar und/oder das Methan in der Waschflüssigkeit zur Bewegung anregbar ist, gekennzeichnet. Damit werden die Methanmoleküle beziehungsweise die Methananhäufungen in der Waschflüssigkeit gut aus den Clusterbindung des Wassers gelockert, wohingegen das in der Waschflüssigkeit enthaltene Kohlendioxid nicht oder weniger angeregt wird. Durch die auf die Waschflüssigkeit einwirkenden elektromagnetischen Wellen beziehungsweise durch elektromagnetisches Wechselfeld werden die dort enthaltenen Methanmoleküle oder Anhäufungen davon angeregt, wie beispielsweise in Schwingungen versetzt. Es werden ein Methanmolekül oder Anhäufungen davon umschießende Wassercluster aufgebrochen beziehungsweise die Bindungskräfte von Methan im Wasser geschwächt, was bewirkt, dass Methan leichter aus der Waschflüssigkeit entweichen kann, so dass aus der mit den elektromagnetischen Wellen beziehungsweise elektromagnetischem Wechselfeld behandelten Waschflüssigkeit zu einem höheren Prozentsatz als im in der Waschflüssigkeit enthalten Gasgemisch Methan entgast werden kann. Der verbleibende Anteil des Methans in dem in der Waschflüssigkeit weiter enthaltenen Gasgemisch ist dann geringer. Somit ist bei der späteren Desorption des Gasgemisches aus der Waschflüssigkeit der Anteil des Methans im Abgas verringert. Dieses ist umwelttechnisch und /oder emissionsrechtlich vorteilhaft. Eine kostenintensive und energieintensive Nachbehandlung des Abgases hinsichtlich Methans ist nicht mehr nötig und das durch das erfindungsgemäße Verfahren entgaste Methan kann beispielsweise zu Heizzwecken oder in Gasmotoren energie- und kostensparend weiterverwendet werden.

In den Unteransprüchen sind vorteilhafte Ausgestaltungen, Weiterbildungen und Verbesserungen des jeweiligen Gegenstandes der Erfindung angegeben.

Besonders gut ist zumindest eine Frequenz der elektromagnetischen Wellen beziehungsweise des elektromagnetischen Wechselfeldes im Bereich von 1,2 bis 4,4 Megahertz wobei vorteilhaft ganz besonders der Frequenzbereiche von 1,7 bis 2,0 Megahertz oder der Bereich von 2,7 bis 3,0 Megahertz herausragt. Daraus folgt jeweils ein besonders hoher Anteil an Methan am durch das Verfahren entgasten Gas beziehungsweise Gasgemisch.

Gemäß einer vorteilhaften Weiterbildung des Verfahrens wirkt auf die Waschflüssigkeit beim Einwirken der elektromagnetischen Wellen beziehungsweise des elektromagnetischen Wechselfeldes auf die Waschflüssigkeit ein statischer Druck von über einem bar, wobei ein statischer Druck von zwei bis vier bar besonders vorteilhaft ist. Dadurch ist die Effizienz der selektiven Entgasung des Methans aus der Waschflüssigkeit besonders hoch.

Gemäß einer weiteren vorteilhaften Weiterbildung des Verfahrens wird für Methan katalytisches Material wie insbesondere Nickel in Kontakt mit der Waschflüssigkeit verwendet. Beispielsweise durch die Verwendung von hochreinem Nickel wird zusätzlich eine katalytische Wirkung bei der Reaktion CO2 + 4H2 → CH4 + 2H2O erreicht, welche den Methananteil im entgasten Gasgemisch zusätzlich erhöht. Besonders gut für diesen zusätzlichen katalytischen Verfahrensteil sind in der Waschflüssigkeit befindliche das katalytische Material enthaltende Elektroden zur Erzeugung der elektromagnetischen Wellen beziehungsweise des elektromagnetischen Wechselfeldes. Vorzugsweise erfolgt während oder nach dem Verfahrensschritt des Einwirkens von elektromagnetischen Wellen beziehungsweise von elektromagnetischem Wechselfeld als Verfahrensschritt das Absenken des Drucks auf die Waschflüssigkeit.

Gemäß einer vorteilhaften Weiterbildung des Verfahrens erfolgt der weitere Verfahrensschritt des Auffangens des aus der Waschflüssigkeit entgasten methanhaltigen Gases beziehungsweise methanhaltigen Gasgemisches. Vorzugsweise wird in einem weiteren Verfahrensschritt das aufgefangene methanhaltige Gas beziehungsweise methanhaltige Gasgemisch zu Heizzwecken oder alternativ zur erneuten Druckwasserwäsche weitergeleitet. Dadurch wird energie- und kostensparend das entgaste methanhaltige Gas beziehungsweise methanhaltige Gasgemisch weiterverwendet. Durch erneute Druckwasserwäsche des entgasten methanhaltigen Gases beziehungsweise Gasgemisches wird die Effizienz der Methangewinnung aus Rohbiogas erhöht.

Vorzugsweise ist das Verfahren hinter dem Absorptionsschritt und vor dem Desorptionsschritt innerhalb eines Druckwasserwäscheverfahrens für Biogasanalgen zwischengeschaltet. Gerade bei der Druckwasserwäsche in Biogasanlagen ist die kostengünstige und energieeffiziente Reduzierung des Methananteils in der Waschflüssigkeit und damit in den Abgasen ein wesentlicher Fortschritt. Vorteilhaft ist der Verfahrensschritt der Absenkung des Drucks auf die Waschflüssigkeit in dem letzten Flashkolonnenentgasungsschritt vor dem Desorptionsschritt des Druckwasserwäscheverfahrens enthalten. Damit fallen ein Schritt eines bekannten Druckwasserwäscheverfahrens und des erfindungsgemäßen Verfahrens zusammen, was Kosten und Aufwand einspart.

Schließlich können die Merkmale der Unteransprüche für das erfindungsgemäße Verfahren im Wesentlichen frei miteinander und nicht durch die in den Ansprüchen vorliegende Reihenfolge festgelegt kombiniert werden, sofern sie unabhängig voneinander sind.

Durch eine Vorrichtung zur Durchführung eines erfindungsgemäßen Verfahren zur Entgasung von Methan aus einer auch Kohlendioxid enthaltenden überwiegend aus Wasser bestehenden Waschflüssigkeit, wie insbesondere bei einer Druckwasserwäsche, mit einem Behälter mit mindestens einer Einfluss- und mindestens einer Ausflussöffnung für Waschflüssigkeit und mit mindestens einer Einrichtung geeignet zum Emittieren von elektromagnetischen Wellen beziehungsweise zum Erzeugen von elektromagnetischem Wechselfeld innerhalb eines Frequenzbereichs von 1,7 bis 2,0 Megahertz und/oder von 2,7 bis 3,0 Megahertz sowie geeignet zur Einwirkung auf zumindest einen Teil bei im Behälter befindlicher Waschflüssigkeit, wird die Aufgabe hinsichtlich einer Vorrichtung gelöst. Mit einer solchen

Vorrichtung werden die oben zum erfindungsgemäßen Verfahren beschriebenen Vorteile erzielt. In diesen Frequenzbereichen befinden sich Frequenzen, die sehr gut selektiv die Methanmoleküle beziehungsweise die Methananhäufungen in der Waschflüssigkeit aus den Clusterbindung des Wassers lockern, wohingegen das in der Waschflüssigkeit enthaltene Kohlendioxid nicht oder weniger angeregt wird.

Vorzugsweise enthält die Vorrichtung eine Steuereinheit zur Steuerung der zumindest einen Einrichtung zum Emittieren von elektromagnetischen Wellen beziehungsweise zum Erzeugen von elektromagnetischem Wechselfeld. Mit der Steuereinheit können die unterschiedlichen Behandlungen der Waschflüssigkeit aufeinander abgestimmt und flexibel je nach Bedarf gesteuert werden, wie beispielsweise die Frequenz, Intensität und Dauer der Behandlung mit elektromagnetischen Wellen beziehungsweise mit elektromagnetischem Wechselfeld.

Gemäß einer vorteilhaften Weiterbildung enthält die Vorrichtung eine Analyseeinheit zur Analyse der Waschflüssigkeit und / oder des entgasten methanhaltigen Gases beziehungsweise methanhaltigen Gasgemisches. Damit können Informationen, wie der Methananteil ermittelt werden, anhand derer die Effektivität der Vorrichtung festgestellt und gegebenenfalls optimiert werden kann. Die analysierten Informationen sind bei der Steuerung der Vorrichtung von Vorteil.

Gemäß einer weiteren vorteilhaften Ausgestaltung umfasst die Einrichtung zum Emittieren von elektromagnetischen Wellen beziehungsweise zum Erzeugen von elektromagnetischem Wechselfeld Elektroden zur Einwirkung auf die Waschflüssigkeit. Damit lässt sich besonders gut ein großes Volumen von Waschflüssigkeit mit einem fluktuierenden elektrischen Feld behandeln. Eine weitere Verbesserung sind Elektroden mit für Methan katalytischem Material wie insbesondere Nickel, denn damit wird die Methanausbeute mit der Vorrichtung noch erhöht, was vor allem bei Anlegen einer entsprechend dem Nickel frequentierter Spannung besonders effektiv ist.

Vorzugsweise enthält die erfindungsgemäße Vorrichtung eine Einrichtung zum Absenken des Drucks auf die Waschflüssigkeit. Diese kann zum Beispiel einen Ultraschallerzeuger umfassen, mittels dessen Ultraschall in die mit elektromagnetischen Wellen beziehungsweise mit elektromagnetischem Wechselfeld behandelten Waschflüssigkeit eingekoppelt wird, und in den Unterdruckphasen des Ultraschalls den Druck auf die Waschflüssigkeit absenkt, was bis hin zur Kavitation in der Waschflüssigkeit führen kann, bei der bekanntlich in einer Flüssigkeit enthaltene Gase besonders gut entgast werden. Nach einer anderen vorteilhaften Variante umfasst die Einrichtung zum Absenken des Drucks auf die

Waschflüssigkeit eine der Behandlung mit elektromagnetischen Wellen beziehungsweise elektromagnetischem Wechselfeld nachgeschaltete Flashkolonne. Dieses ist bekanntlich ein besonders gutes Mittel zur Entgasung von Flüssigkeiten.

Gemäß einer vorteilhaften Weiterbildung enthält die Vorrichtung eine Gasauffangeinrichtung zum Auffangen des mit der Vorrichtung aus der Waschflüssigkeit entgasten Gases. Dieses Methan enthaltende Gas beziehungsweise Gasgemisch kann dann vorteilhaft weiterverwendet werden, wie beispielsweise zu Heizzwecken oder in Gasmotoren oder erneut in der Druckwasserwäsche.

Gemäß einer vorteilhaften Ausgestaltung ist die Vorrichtung oder mehrere davon in eine Druckwasserwaschanlage einer Biogasanlage nach der Absorptionseinrichtung und vor der Desorptionseinheit integrierbar ausgestaltet. Gerade bei der Druckwasserwäsche in Biogasanlagen ist die kostengünstige und energieeffiziente Reduzierung des Methananteils in der Waschflüssigkeit und damit in den Abgasen ein wesentlicher Fortschritt. Eine weitere Verbesserung ist eine Integration der mindestens einen Vorrichtung mit einer Bypassschaltung in einer Druckwasserwaschanlage bei der Reinigung der Waschflüssigkeit, so dass die Vorrichtung/en bei der Reinigung der Waschflüssigkeit je nach Bedarf zu- und abschaltbar ist/sind, was eine besonders flexible und damit energiesparende Handhabung der Vorrichtung/en innerhalb der Druckwasserwaschanlage ermöglicht.

Schließlich können die Merkmale der Unteransprüche für das erfindungsgemäße Vorrichtung im Wesentlichen frei miteinander und nicht durch die in den Ansprüchen vorliegende Reihenfolge festgelegt kombiniert werden, sofern sie unabhängig voneinander sind.

Ein Ultraschallerzeuger ist üblicherweise ein elektrisch betriebener Ultraschallwandler, wie beispielsweise eine Sonotrode.

Entsprechend wird die Aufgabe durch die Verwendung einer erfindungsgemäßen Vorrichtung und/oder einer Vorrichtung, die einen Behälter mit mindestens einer Einflussöffnung und mindestens einer Ausflussöffnung für Waschflüssigkeit und mindestens eine Einrichtung geeignet zum Emittieren von elektromagnetischen Wellen beziehungsweise zum Erzeugen von elektromagnetischem Wechselfeld innerhalb eines Frequenzbereichs von 0,5 bis 10 Megahertz sowie geeignet zur Einwirkung auf zumindest einen Teil bei im Behälter befindlicher Waschflüssigkeit enthält, zur Entgasung von Methan aus einer auch Kohlendioxid enthaltenden überwiegend aus Wasser bestehenden Waschflüssigkeit, wie insbesondere bei einer Druckwasserwäsche, gelöst.

### Kurze Beschreibung der Zeichnungen:

Anhand der Zeichnungen werden Ausführungsbeispiele der Erfindung erläutert.

Es zeigen
Figur 1 ein Flussdiagramm eines Ausführungrsbeispiels des erfindungsgemäßen Verfahrens,
Figur 2 eine Blockdarstellung einer Druckwasserwäscheanlage mit einem Ausführungsbeispiel der Erfindung,
Figur 3 eine Blockdarstellung einer Druckwasserwäscheanlage mit einem weiteren Ausführungsbeispiel der Erfindung,
Figur 4a im Querschnitt entlang der Längsachse und senkrecht zu enthaltenen in Plattenform ausgestalteten Elektroden ein Ausführungsbeispiel der erdfindungsgemäßen Vorrichtung,
Figur 4b im Querschnitt entlang der Längsachse und parallel zu enthaltenen in Plattenform ausgestalteten Elektroden das gleiche Ausführungsbeispiel der erfindungsgemäßen Vorrichtung,
Figur 5 im Querschnitt entlang der Längsachse ein weiteres Ausführungsbeispiel der erdfindungsgemäßen Vorrichtung, und
Figur 6 im Querschnitt entlang der Längsachse schematisch eine gemäß dem Stand der Technik übliche Flashkolonne.

### Ausführliche Beschreibung der Erfindung:

Alle Zeichnungen sind schematisch zu verstehen. Auf maßstabsgetreue Abbildungen wurde zum Zwecke erhöhter Klarheit der Darstellung verzichtet.

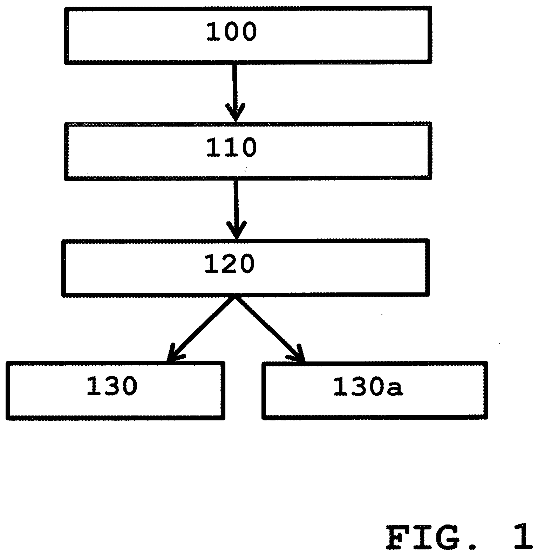

Figur 1 zeigt ein Flussdiagramm eines Ausführungsbeispiels des erfindungsgemäßen Verfahrens. Dabei wird vorwiegend aus Wasser bestehende Waschflüssigkeit, wie beispielsweise aus einer Druckwasserwäsche von Rohbiogas, in einen Behälter geleitet. Die Waschflüssigkeit enthält das aus dem Rohbiogas ausgewaschene Kohlendioxid und kleine Mengen Methan von ca. 0,5% bis ca. 2% des ausgewaschenen Gases. Im ersten Verfahrensschritt 100 wird die in einen Behälter eingeleitete Waschflüssigkeit elektromagnetischen Wellen beziehungsweise elektromagnetischem Wechselfeld ausgesetzt. Dieses geschieht durch eine Einrichtung zum Emittieren von elektromagnetischen Wellen beziehungsweise elektromagnetischem Wechselfeld, wie beispielsweise über ein fluktuierendes elektrisches Feld erzeugende Elektroden an der Waschflüssigkeit. Die Frequenz der elektromagnetischen Wellen beziehungsweise elektromagnetischem Wechselfeld liegt im Frequenzbereich, in dem durch das Einwirken die Bindung des Methans in der Waschflüssigkeit verringerbar und/oder das Methan in der Waschflüssigkeit zur Bewegung anregbar ist, wie beispielsweise zu Molekülschwingungen. Die Frequenz liegt beispielsweise im Bereich von 0,5 Megahertz bis 10 Megahertz oder beispielsweise für besonders effektive Wirkung im Frequenzbereiche von 1,2 Megahertz bis 4,4 Megahertz. Es sind auch mehrere unterschiedliche Frequenzen mehrerer elektromagnetischer Wellen gleichzeitig denkbar, die jeweils in einem oben beschriebenen hinsichtlich Methan wirkungsvollen Frequenzbereich liegen. Ganz besonders wirkungsvoll ist das Verfahren bei Frequenzen der elektromagnetischen Wellen beziehungsweise des elektromagnetischen Wechselfeldes in den Bereichen von 1,7 bis 2,0 Megahertz und/oder von 2,7 bis 3,0 Megahertz. Der prozentuale Anteil des Methans im entgasten Gasgemisch aus der Waschflüssigkeit einer Druckwasserwäsche lässt sich so im Vergleich zur Entgasung ohne Anwendung des erfindungsgemäßen Verfahrens um ungefähr ein Drittel und je nach Einstellung auch noch mehr erhöhen. Die elektrische Leistung beträgt für das Erzeugen der elektromagnetischen Wellen beziehungsweise elektromagnetischen Wechselfeld beispielsweise 320 Watt aus circa 5,15 Volt Spannung und circa 62 Ampere Stromstärke. Durch Anregung des Methans mittels elektromagnetischer Wellen beziehungsweise elektromagnetischem Wechselfeld wird die Bindung des Methans in der Waschflüssigkeit verringert. Die überwiegend aus Wasser bestehende Waschflüssigkeit ist beispielsweise Wasser, was mit unter den Begriff "überwiegend" zu verstehen ist, oder zum Beispiel mit Zusätzen wie Diethanolamin versetztes Wasser, wobei der Mengenanteil des Wassers noch über der Hälfte liegt, also der Anteil von Zusätzen zum Wasser in der Waschflüssigkeit im Bereich von Null bis zur Grenze, bei der noch gerade der Anteil des Wassers über die Hälfte ausmacht, liegen kann. Hingegen wird das auch in der Waschflüssigkeit enthaltene Kohlendioxid nicht durch die eingestrahlte elektromagnetische Welle beziehungsweise elektromagnetischem Wechselfeld angeregt, denn deren Anregungsfrequenzen liegen in einem anderen Frequenzbereich. Das Kohlendioxid ist in der Waschflüssigkeit stärker als das angeregte Methan gebunden. So existiert bei Kohlendioxid eine stärkere Dipol-Dipol-Verbindung mit dem Wasser und teilweise ist das Kohlendioxid CO₂ mit dem Wasser sogar zu Kohlensäure H2CO3 reagiert. Auf der Waschflüssigkeit wirkt im ersten Verfahrensschrift 100 ein statischer Druck von zwei bis vier bar. Denkbar ist auch ein anderer statischer Druck, wie beispielsweise Normaldruck oder etwas mehr als vier bar. Ein weiterer optionaler Verfahrensteil im Verfahrensschritt 100 ist die Katalyse der Reaktion von in der Waschflüssigkeit vorhandenem Kohlendioxid und Wasserstoff in der Waschflüssigkeit zu Methan und Wasser durch katalytisches Material wie hochreines Nickel in Kontakt mit den in Plattenform ausgestalteten Elektroden für das fluktuierende elektromagnetische Feld. Denkbar ist eine Optimierung der Wirkung des Nickels durch das Anlegen einer entsprechend dafür dem Nickel frequentierten Spannung. Im folgenden optionalen Verfahrensschritt 110 wird der Druck auf die Waschflüssigkeit abgesenkt. Dieses erfolgt beispielsweise in einer nachgeschalteten Flashkolonne. Auch andere Methoden zur Absenkung des Drucks sind denkbar, wie beispielsweise die wiederholt phasenweise Druckabsenkung durch in die Waschflüssigkeit eingekoppelten Schall. So ist durch ausreichend großen Ultraschall Kavitation in der Waschflüssigkeit möglich, die eine Entgasung des durch die Anregung mit elektromagnetischen Wellen beziehungsweise elektromagnetischen Wechselfeld in der Bindung in der Waschflüssigkeit gelockerten Methans fördert. Es ist auch denkbar, dass die beiden Verfahrensschritte 100 und 110 statt nacheinander gleichzeitig stattfinden.

Im optionalen, also nicht zwingend vorgesehenen, aber vorteilhaften dritten Verfahrensschritt 120 wird das aus der Waschflüssigkeit entgaste methanhaltige Gas beziehungsweise Gasgemisch aufgefangen und im weiteren optionalen Verfahrensschritt 130 zur weiteren Verwendung wie beispielsweise zu Heizzwecken oder in Gasmotoren oder wieder im optional Verfahrensschritt 130a zum erneuten Durchlauf des Reinigungsverfahren, wie beispielsweise einer Druckwasserwäsche, des Gases weitergeleitet. Die Waschflüssigkeit wird nach dem erfindungsgemäßen Entgasen des Methans weiter zur Reinigung beziehungsweise Desorption insbesondere vom in ihr noch gebundenen Kohlendioxid geleitet. Das erfindungsgemäße Verfahren ist zum Beispiel hinter dem Absorptionsschritt und vor dem Desorptionsschritt innerhalb eines Druckwasserwäscheverfahrens für Biogasanlagen zwischengeschaltet. Dabei ist eine Variante der Verfahrensschritt 110 der Absenkung des Drucks auf die Waschflüssigkeit im Flashkolonnenentgasungsschritt vor dem Desorptionsschritt des Druckwasserwäscheverfahrens.

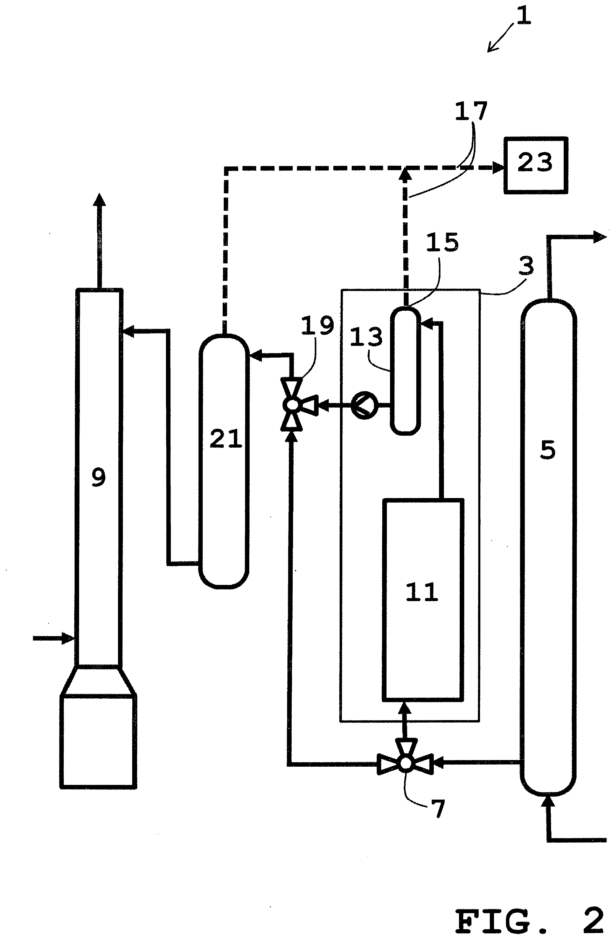

In Figur 2 ist eine Blockdarstellung einer Druckwasserwaschanlage 1 mit einem Ausführungsbeispiel der Erfindung 3 gezeigt. Das Rohgas, beispielsweise einer Biogasanlage mit circa 52 % Methan und circa 48 % Kohlendioxid, wird in der Absorptionsstufe 5 unter Druck von ungefähr sieben bar im Gegenstromverfahren mit Waschflüssigkeit, die entweder zu einem großen Teil aus Wasser oder ganz aus Wasser besteht, gereinigt. Dabei nimmt die Waschflüssigkeit vorwiegend das im Rohgas enthaltene Kohlendioxid auf, also wäscht dieses aus dem Rohgas aus, so dass als Gas gereinigtes Methan von ca. 98% Reinheit übrig bleibt, welches dann gut nutzbar ist. In der Absorptionsstufe 5 enthält jedoch auch das in der Waschflüssigkeit ausgewaschene Gasgemisch zu circa 0,5% bis circa 2% Methan. Nach der Absorptionsstufe 5 wird die Waschflüssigkeit von ungefähr sieben bar statischen Druck auf im Bereich von circa zwei bis circa vier bar statischen Druck durch eine übliche Entspannungseinrichtung wie beispielsweise ein Expansionsventil entspannt und zu einem Dreiwegehahn 7 geleitet, an dem je nach Bedarf die Waschflüssigkeit entweder nach einer Ausführungsform der erfindungsgemäßen Vorrichtung 3 zur Durchführung des erfindungsgemäßen Verfahrens oder gleich einer Flashkolonne 21 der Druckwasserwäscheanlage 1 zugeführt wird. Beispielsweise durch vorgeschaltete Analyse des Methananteils in der Waschflüssigkeit kann entschieden werden, ob die als Bypassschaltung in die Druckwasserwäscheanlage 1 eingebundene erfindungsgemäße Vorrichtung 3 zur selektiven Methanentgasung eingesetzt wird oder nicht. Soweit aus der Waschflüssigkeit selektiv Methan entgast werden soll, wird diese vom Dreiwegehahn 7 aus in einen Behälter 11 geleitet. An oder in diesem Behälter 11 befindet sich mindestens eine Einrichtung zum Emittieren von elektromagnetischen Wellen beziehungsweise von elektromagnetischem Wechselfeld innerhalb eines Frequenzbereiches, in dem durch das Einwirken die Bindung des Methans in der Waschflüssigkeit verringerbar und/oder das Methan in der Waschflüssigkeit zur Bewegung anregbar ist.

So wirken beispielsweise elektromagnetische Wellen beziehungsweise elektromagnetisches Wechselfeld mit Frequenzen von 1,85 Megahertz und 2,85 Megahertz anregend auf das Methan in der Waschflüssigkeit, aber auch bei elektromagnetische Wellen beziehungsweise elektromagnetisches Wechselfeld in anderen Frequenzbereichen wie beispielsweise 39 Kilohertz ist die Bindung des Methans in der Waschflüssigkeit verringerbar. Die elektromagnetischen Wellen beziehungsweise elektromagnetische Wechselfeld wirken auf zumindest einen Teil der Waschflüssigkeit vorzugweise die gesamte Waschflüssigkeit im Behälter 11 ein. Diese Einrichtung enthält Elektrodenpaare aus hochreinem Nickel, an denen das auf die Waschflüssigkeit fluktuierende elektromagnetische Feld also das elektromagnetische Wechselfeld erzeugt wird. Denkbar ist auch ein geeignet starker Radiowellenemitter zu Erzeugung der einwirkenden elektromagnetischen Wellen. Ein solcher Behälter 11 mit der Einrichtung zur Emittierung der elektromagnetischen Wellen beziehungsweise elektromagnetischem Wechselfeld wird auch als EmF-Reaktor bezeichnet. Eine elektrische Leistung von ungefähr 320 Watt ist bereits ausreichend für einen signifikanten Effekt für die selektive Entgasung von Methan aus der Waschflüssigkeit. Unmittelbar danach gelangt die so behandelte Waschflüssigkeit in eine zu diesem Ausführungsbeispiel der erfindungsgemäßen Vorrichtung 3 gehörende Flashkolonne 13, in der der auf die Waschflüssigkeit herrschende Druck abgesenkt wird. Die Flashkolonne 13 ist wie eine übliche Flashkolonne aufgebaut. In ihr 13 wird die Waschflüssigkeit entgast. Auch bereits im EmF-Reaktor 11 aus der Waschflüssigkeit ausgetretenes methanhaltiges Gas beziehungsweise Gasgemisch wird dabei von der Waschflüssigkeit getrennt. Das methanhaltige Gas beziehungsweise Gasgemisch wird im oberen Bereich 15 der Flashkolonne 13 aufgefangen und durch die oben aus der Flashkolonne 13 geführte Leitung 17 zur Gasheizung 23 geführt. Es sind auch andere Verwendungen des methanhaltigen Gases beziehungsweise Gasgemisches denkbar, wie beispielweise in einem Gasmotor oder die Rückführung in die Rohbiogaszuleitung zur Absorptionsstufe 5 der Druckwasserwäscheanlage 1.

Im unteren Bereich der Flashkolonne 15 wird die überproportional vom Methan entgaste aber noch Kohlendioxid enthaltende Waschflüssigkeit über einen weiteren Dreiwegehahn 19 weiter zur zweiten Flashkolonne 21 geleitet, in der die Waschflüssigkeit weiter entgast wird. Auch das ebenfalls noch Methan enthaltende Gas beziehungsweise Gasgemisch wird aufgefangen und durch eine Leitung zur weiteren Verwendung wie in einer Gasheizung 23 geführt. Die Waschflüssigkeit wird hingegen von der zweiten Flashkolonne 21 zur Desorptionsstufe 11 geleitet. In der Desorptionsstufe 11 wird mittels Luft die Waschflüssigkeit weiter entgast, wobei nunmehr im Wesentlichen das Kohlendioxid aus der Waschflüssigkeit als Abgas entweicht. Dieses Abgas enthält dann fast kein Methan mehr und ist diesbezüglich umwelttechnisch und emissionsrechtlich unbedenklich.

Es ist auch denkbar, dass die erfindungsgemäße Vorrichtung 3 im Wesentlichen nur einen EmF-Reaktor 11 umfasst und die dort behandelte Waschflüssigkeit statt in eine erste Flashkolonne 13 gleich in die letzte Flashkolonne 21 der Druckwasserwäscheanlage 1 geleitet wird. Ferner ist eine Förderung der Entgasung des methanhaltigen Gases beziehungsweise Gasgemisches aus der Waschflüssigkeit bereits während der Behandlung mit den elektromagnetischen Wellen beziehungsweise elektromagnetischem Wechselfeld beispielsweise mit durch eine Sonotrode in die Waschflüssigkeit eingekoppelten. Ultraschall denkbar. In den Unterdruckphasen des Ultraschalls wird das methanhaltige Gas beziehungsweise Gasgemisch aus der Waschflüssigkeit gelöst, was besonders gut bei durch Ultraschall auftretender Kavitation erfolgt.

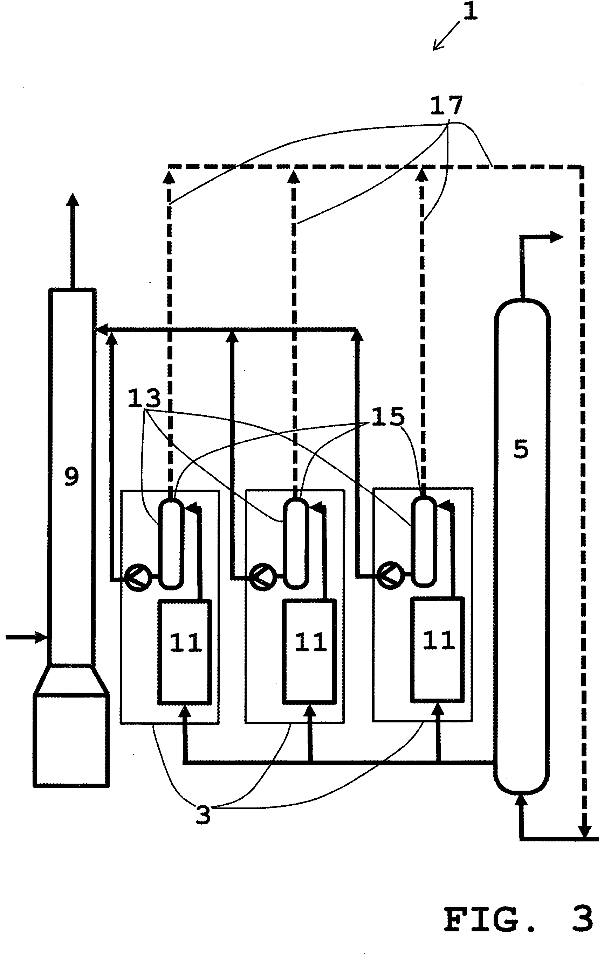

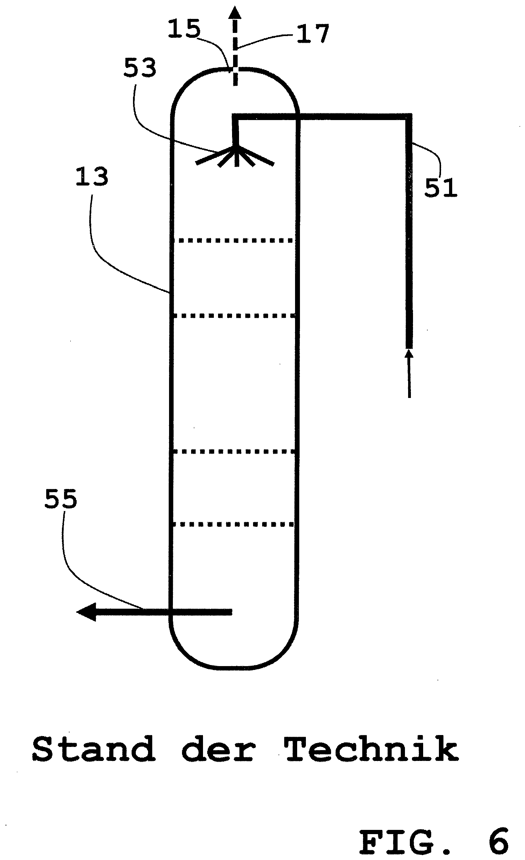

In Figur 3 ist in einer Blockdarstellung eine Druckwasserwäscheanlage 1 mit einem weiteren Ausführungsbeispiel 3 der Erfindung gezeigt. Das Rohbiogas gelangt in die Absorptionsstufe 5, in der mittels Druckwasserwäsche im Wesentlichen das Kohlendioxid ausgewaschen wird. Das übrige hauptsächlich aus Methan bestehende Gasgemisch wird oben zur weiteren Verwendung aus der Absorptionsstufe 5 geleitet. Hingegen wird die zumindest überwiegend aus Wasser bestehende Waschflüssigkeit aus der Absorptionsstufe 5 weiter zu drei parallel geschalteten erfindungsgemäßen Vorrichtungen 3 zur selektiven Entgasung bei erhöhtem statischen Druck von zwei bis vier bar geleitet. Denkbar ist auch eine andere Anzahl von erfindungsgemäßen Vorrichtungen 3, je nach gewünschter Leistung der selektiven Entgasung von Methan aus der Waschflüssigkeit. Denkbar ist dabei auch jeweils eine Bypassschaltung zum Zu- und Abschalten einzelner oder mehrerer erfindungsgemäßer Vorrichtungen 3 je nach Bedarf. Die mit Druck beaufschlagte Waschflüssigkeit gelangt in die EmF-Reaktoren 11 und wird dort mittels nickelhaltiger Elektroden in die Waschflüssigkeit eingekoppelten mit elektromagnetischen Wellen beziehungsweise elektromagnetischem Wechselfeld behandelt. Die Frequenz liegt dabei in einem Frequenzbereich, in dem durch das Einwirken der elektromagnetischen Wellen beziehungsweise des elektromagnetischen Wechselfeldes die Bindung des Methans in der Waschflüssigkeit verringert und/oder das Methan in der Waschflüssigkeit zur Bewegung anregt wird. Beispielsweise Frequenzen von circa 1,85 oder circa 2,85 Megahertz sind verwendbar. Aber auch andere geeigneten Frequenzen insbesondere im Bereich von 0,5 bis 10 Megahertz sind möglich. Die so in den EmF-Reaktoren 11 behandelte Waschflüssigkeit wird unmittelbar durch jeweils eine Leitung daran anschließend in die jeweilige Flashkolonne 13 zum Entgasen durch insbesondere Entspannen des Drucks auf die Waschflüssigkeit geleitet. Ein Ausführungsbeispiel einer dem Stand der Technik entsprechenden Flashkolonne ist schematisch in Figur 6 dargestellt. Das durch die Behandlung im jeweiligen EmF-Reaktor 11 mit einem relativ hohen Methananteil in der jeweiligen Flashkolonne 13 entgaste Gas beziehungsweise Gasgemisch wird im oberen Bereich 15 der jeweiligen Flashkolonne 13 aufgefangen wohingegen die selektiv entgaste Waschflüssigkeit im unteren Bereich der jeweiligen Flashkolonne 13 sammelt und jeweils in Richtung der Desorptionsstufe 9 der Druckwasserwäscheanlage 1 geleitet wird. Dort wird die Waschflüssigkeit vom restlich in ihr enthaltenen Gas gereinigt. Das jedoch bereits in den oberen Bereichen 15 der Flashkolonnen 13 aufgefangene methanhaltige Gas beziehungsweise Gasgemisch wird über die Leitungen 17 zusammengeführt und wieder vor der Absorptionsstufe 5 in die Zuleitung des Rohgases wie beispielsweise Rohbiogases geleitet.

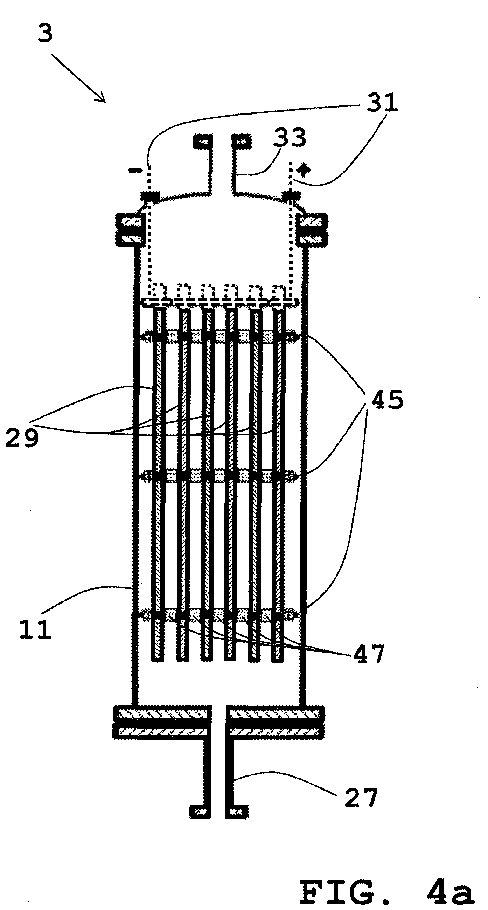

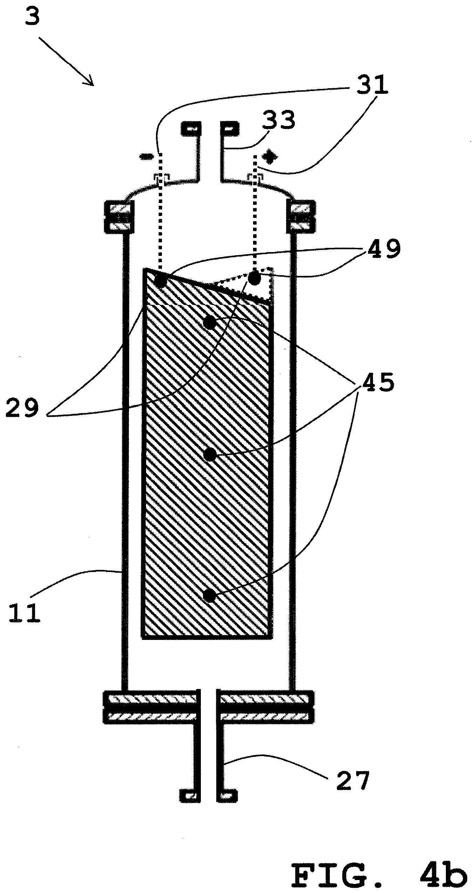

In Figur 4a ist im Querschnitt entlang der Längsachse und senkrecht zu den als Platten enthaltenen Elektroden 29 ein Ausführungsbeispiel der erdfindungsgemäßen Vorrichtung 3 dargestellt. Figur 4b zeigt von der gleichen Vorrichtung 3 im Querschnitt entlang ihrer Längsachse die Ansicht parallel zu enthaltenen Elektroden 29. Die Kohlendioxid und Methan enthaltende Waschflüssigkeit fließt durch die Einflussöffnung 27 in den zylinderförmigen Behälter 11. In diesem Behälter 11 befinden sich mehrere parallel zueinander angeordnete plattenförmige Elektroden 29 aus beispielsweise Nickel. Der Abstand zwischen den plattenförmigen Elektroden 29 beträgt jeweils nur wenige Millimeter wie beispielsweise zwei Millimeter. Jeweils benachbarte plattenförmige Elektroden 29 sind elektrisch isoliert zueinander durch in Figur 4a dargestellte Isolatoren 47 über Halterungen 45 befestigt. Die elektrische Polung von benachbarten plattenförmigen Elektroden 29 ist jeweils entgegengesetzt, wobei diese jeweils durch den jeweiligen in Figur 4b dargestellten Anschluss 49 an die jeweilige der beiden Leitungen 31 elektrisch leitend angeschlossen sind. Die beiden Leitungen 31 führen zu dem Generator, der elektromagnetischen Wechselstroms für das im Behälter 11 zu erzeugende elektromagnetische Wechselfeld liefert, nach oben aus dem Behälter 11 heraus. Diese Leitungen 31 sind in den Figuren 4a und 4b gestrichelt dargestellt, weil sie streng genommen nicht in der jeweiligen Querschnittfläche liegen, sondern davor beziehungsweise dahinter. Frequenzen des elektromagnetischen Wechselfeldes von 1,8449 Megahertz oder 2,8449 Megahertz sind verwendbar. Aber auch andere geeignete Frequenzen, bei denen durch das Einwirken die Bindung des Methans in der Waschflüssigkeit verringerbar und/oder das Methan in der Waschflüssigkeit zur Bewegung anregbar ist, insbesondere aus dem Bereich von 0,5 Megahertz bis 10 Megahertz sind denkbar. Die elektrische Leistung des von den Elektroden 29 aus in die Waschflüssigkeit des Behälters 11 einwirkende elektromagnetische Wechselfeldes beträgt 320 Watt. Auch höhere, sogar deutlich höhere Leistungen sind denkbar. Durch die Einwirkung des elektromagnetischen Wechselfeldes auf die Waschflüssigkeit kommt es bereits zur ersten Entgasung von vorzugsweise Methan CH₄ aus der Waschflüssigkeit. Dieses Gasgemisch und die Waschflüssigkeit strömen nach der Behandlung durch das elektromagnetische Wechselfeld durch die Ausflussöffnung 33 oben aus dem Behälter 11 hinaus. Danach wird das entgaste methanhaltige Gasgemisch durch übliche Mittel wie beispielsweise in einer Flashkolonne von der Waschflüssigkeit getrennt und eventuell noch die Entgasung der Waschflüssigkeit weiter fortgesetzt.

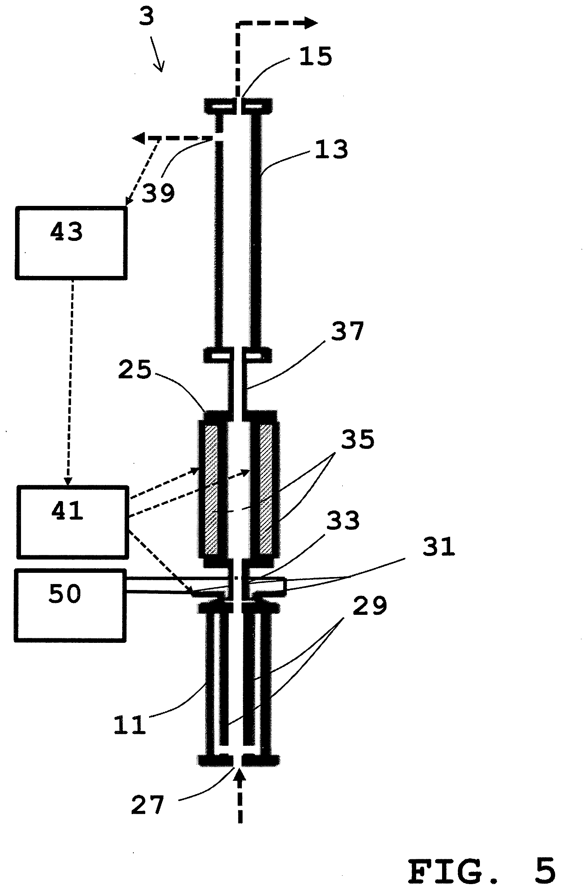

In Figur 5 ist im Querschnitt entlang der Längsachse ein Ausführungsbeispiel der erfindungsgemäßen Vorrichtung 3 dargestellt. Die Waschflüssigkeit gelangt durch die Einflussöffnung 27 in den Behälter 11. In dem auch als EmF-Reaktor bezeichneten Behälter 11 befinden sich Elektroden 29. Diese sind beispielsweise mehrere Platten, die parallel zueinander angeordnet sind und einen Abstand der parallelen Flächen von nur wenigen Millimetern, wie zum Beispiel vier Millimetern, aufweisen. Benachbarte plattenförmige Elektroden 29 haben dabei entgegengesetzte elektrische Polung, die durch entsprechende Kontakte 31 zu einer von außen angeschlossenen elektrische Wechselspannung in der gewünschten und geeigneten Frequenz erzeugenden Stromquelle 50 verbunden sind. Die Elektroden 29 bestehen aus einem geeigneten Material, wie hier aus Nickel, aber auch andere Materialien wie beispielsweise Zinn, Kupfer oder Graphit sind denkbar. Das zwischen den Elektroden 29 erzeugte fluktuierende elektromagnetische Feld wirkt als elektromagnetische Wellen auf die dort befindliche Waschflüssigkeit ein. Die Frequenz des elektromagnetischen Wechselfeldes wird dabei so gewählt, dass das in der Waschflüssigkeit enthaltene Methan zu Bewegungen angeregt wird beziehungsweise die Bindung des Methans in der Waschflüssigkeit verringert wird. Hierzu reicht beispielsweise bereits eine Frequenz von 1,8 Megahertz um eine die Bindung in der Waschflüssigkeit verringernde Wirkung auf das in der Waschflüssigkeit enthaltene Methan festzustellen. Aber auch bei spezifischen höheren Frequenzen der elektromagnetischen Wellen beziehungsweise des elektromagnetischen Wechselfeldes wie beispielsweise circa 2,85 Megahertz wird eine signifikante vorteilhafte Erhöhung des Methanteils des Gasgemisches bei der späteren Entgasung in den beiden folgenden Behältern 25 und 13 erreicht. Mittels der Steuereinheit 41 lässt sich die Spannung, Stromstärke und Frequenz der Fluktuation des an den Elektroden 29 erzeugten elektromagnetischen Wechselfeldes steuern und auf die Anregung des in der Waschflüssigkeit enthaltenen Methans für dessen bevorzugte beziehungsweise selektive insbesondere später in den Behältern 25 und 13 erfolgende Entgasung optimiert einstellen. Durch die Verwendung von hochreinem Nickel als Material auf den Elektrodenoberflächen wird zusätzlich eine katalytische Wirkung bei der Reaktion

CO2 + 4H2 → CH4 + 2H2O

erreicht, welche den Methananteil im später in den Behälter 25 und 13 entgasten Gasgemisch zusätzlich erhöht. Die im EmF-Reaktor 11 elektromagnetisch behandelte Waschflüssigkeit fließt durch die Ausflussöffnung 33 in den Behälter 25. An diesem Behälter 25 sind als Ultraschallerzeuger außen Sonotroden 35 angebracht, mit denen Ultraschall erzeugt wird, der in die im Behälter 25 befindliche Waschflüssigkeit eingekoppelt wird. Die Frequenz des so erzeugten Ultraschalls liegt in diesem Fall im Bereich zwischen 18 und 30 Kilohertz. Denkbar sind aber auch Ultraschallfrequenzen von über 30 Kilohertz. Eine Ausführungsform mit nur einer einzigen Sonotrode 35 ist denkbar. Auch Sonotroden, die im direkten Kontakt zur Waschflüssigkeit stehen, wie beispielsweise in den Behälter 25 eingelassene Stabsonotroden, sind für die Erzeugung und Einkopplung des Ultraschalls in die Waschflüssigkeit möglich. Die Schallamplitude des in die Waschflüssigkeit eingekoppelten Ultraschalls ist ausreichend hoch, um zumindest in Teilbereichen der im Behälter 25 befindlichen Waschflüssigkeit Kavitation beziehungsweise Bläschen zu erzeugen. Eine ausreichend hohe Schallamplitude hängt insbesondere vom in der Waschflüssigkeit herrschenden statischen Druck und der Temperatur ab. Je höher der statische Druck ist, desto größer muss die eingekoppelte Schallamplitude des Ultraschalls sein, um Kavitation beziehungsweise Bläschen zu erzeugen. Um ausreichenden Unterdruck in der Unterdruckphase des Ultraschalls für Kavitation zu erreichen, muss dem vorhandenen statischen Druck dabei entgegengewirkt werden. Durch die Kavitation beziehungsweise Bläschenbildung wird die Entgasung von in der Waschflüssigkeit enthaltenen Gasbestandteilen, wie insbesondere Methan, gefördert. Die Höhe und Frequenz des Schalldrucks des Ultraschalls lässt sich mittels der Steuereinheit 41 zu den Sonotroden 35 steuern und optimiert auf die Entgasung von methanhaltigem Gas beziehungsweise Gasgemisch aus der Waschflüssigkeit einstellen. Die so mit Ultraschall behandelte Waschflüssigkeit sowie der bereits entgaste Teil des methanhaltigen Gases beziehungsweise Gasgemisches strömen durch die Ausfluss- beziehungsweise Einflussöffnung 37 in die Flashkolonne 13. Dort wird die Entgasung der Waschflüssigkeit nach dem für eine Fashkolonne bekannten Stand der Technik fortgeführt und die selektiv hinsichtlich bevorzugt Methan entgaste Waschflüssigkeit zur weiteren Behandlung durch die Ausflussöffnung 39 aus dem als Flashkolonne ausgestalteten Behälter 13 beziehungsweiser der gesamten Vorrichtung 3 herausgeleitet. Diese Waschflüssigkeit wird dort mittels einer Analyseeinheit 43 insbesondere auf die in ihr enthaltenen beziehungsweise gelösten Gase wie Methan und Kohlendioxid und deren Mengenverhältnis hin analysiert und diese gewonnenen Informationen zur Steuereinheit 41 übermittelt, die 41 diese Informationen zur Einstellung der Sonotroden 35 für den Ultraschall und der Einrichtungen 29 zum Emittieren der elektromagnetischen Wellen beziehungsweise des elektromagnetischen Wechselfeldes verwendet. Das aus der Waschflüssigkeit entgaste Gas beziehungsweise Gasgemisch, das Methan enthält, wird im oberen Bereich 15 der Flashkolonne 13 aufgefangen und durch eine obere Öffnung zur weiteren Verwendung beispielsweise in einem Gasmotor geleitet.

In Figur 6 ist im Querschnitt entlang der Längsachse schematisch eine gemäß dem Stand der Technik übliche Flashkolonne 13 dargestellt. Über die Zuleitung 51 wird Gas enthaltene und mit Druck beaufschlagte Waschflüssigkeit in die Flashkolonne 13 geleitet und durch eine Art Duschkopf 53 von oben in das Innere der Flashkolonne 13 ausgesprüht, wobei der danach auf die Waschflüssigkeit herrschende Druck in der Flashkolonne deutlich geringer ist. Im oberen Bereich 15 der Flashkolonne 13 wird das dabei aus der Waschflüssigkeit entgaste Gas beziehungsweise Gasgemisch aufgefangen und durch eine obere Öffnung durch die Leitung 17 zur weiteren Verwendung aus der Flashkolonne 13 herausgeführt. Die im unteren Bereich der Flashkolonne 13 sich sammelnde entgaste und unter jetzt geringerem Druck stehende Waschflüssigkeit fließt durch die Leitung 55 aus der Flashkolonne 13 heraus.

## Patentansprüche

1. Verfahren zur Entgasung von Methan aus einer auch Kohlendioxid enthaltenden überwiegend aus Wasser bestehenden Waschflüssigkeit, wie insbesondere bei einer Druckwasserwäsche, **gekennzeichnet durch**
Einwirken von elektromagnetischen Wellen beziehungsweise von elektromagnetischem Wechselfeld auf die Waschflüssigkeit mit zumindest einer Frequenz innerhalb eines Frequenzbereichs von 0,5 bis 10 Megahertz, wobei bei der zumindest einen Frequenz durch das Einwirken die Bindung des Methans in der Waschflüssigkeit verringerbar und/oder das Methan in der Waschflüssigkeit zur Bewegung anregbar ist (100).

2. Verfahren nach Anspruch 1, wobei zumindest eine Frequenz der elektromagnetischen Wellen beziehungsweise des elektromagnetischen Wechselfeldes innerhalb eines Frequenzbereichs von 1,7 bis 2,0 Megahertz oder von 2,7 bis 3,0 Megahertz liegt.

3. Verfahren nach Anspruch 1 oder 2, wobei beim Einwirken der elektromagnetischen Wellen beziehungsweise des elektromagnetischen Wechselfeldes auf die Waschflüssigkeit ein statischer Druck von über einem bar auf die Waschflüssigkeit wirkt.

4. Verfahren nach Anspruch 3, wobei beim Einwirken der elektromagnetischen Wellen beziehungsweise des elektromagnetischen Wechselfeldes auf die Waschflüssigkeit ein statischer Druck von zwei bis vier bar wirkt.

5. Verfahren nach einem der Ansprüche 1 bis 4, wobei für Methan katalytisches Material wie insbesondere Nickel in Kontakt mit der Waschflüssigkeit verwendet wird.

6. Verfahren nach einem der Ansprüche 1 bis 5 mit dem während oder nach dem Verfahrensschritt (100) des Einwirkens von elektromagnetischen Wellen beziehungsweise von elektromagnetischem Wechselfeld erfolgenden Verfahrensschritt (110) des Absenkens des Drucks auf die Waschflüssigkeit.

7. Verfahren nach einem der Ansprüche 1 bis 6 mit dem weiteren Verfahrensschritt (120) des Auffangens des aus der Waschflüssigkeit entgasten methanhaltigen Gases beziehungsweise methanhaltigen Gasgemisches.

8. Verfahren nach Anspruch 7 mit dem weiteren Verfahrensschritt des Weiterleitens (130a) des aufgefangenen methanhaltigen Gases beziehungsweise methanhaltigen Gasgemisches zur erneuten Behandlung in einer Druckwasserwäsche.

9. Verfahren nach einem der Ansprüche 1 bis 8, wobei es hinter dem Absorptionsschritt und vor dem Desorptionsschritt innerhalb eines Druckwasserwäscheverfahrens für Biogasanlagen zwischengeschaltet ist.

10. Verfahren nach Anspruch 9, wobei der Verfahrensschritt (110) der Absenkung des Drucks auf die Waschflüssigkeit in dem letzten Flashkolonnenentgasungsschritt vor dem Desorptionsschritt des Druckwasserwäscheverfahrens enthalten ist.

11. Vorrichtung (3) zur Durchführung eines Verfahrens nach einem der Ansprüche 1 bis 10 zur Entgasung von Methan aus einer auch Kohlendioxid enthaltenden überwiegend aus Wasser bestehenden Waschflüssigkeit, wie insbesondere bei einer Druckwasserwäsche, **dadurch gekennzeichnet,**
- **dass** sie (3) einen Behälter (11) mit mindestens einer Einflussöffnung (27) und mindestens einer Ausflussöffnung (33) für Waschflüssigkeit enthält,
- **dass** sie (3) mindestens eine Einrichtung (29, 31) geeignet zum Emittieren von elektromagnetischen Wellen beziehungsweise zum Erzeugen von elektromagnetischem Wechselfeld innerhalb eines Frequenzbereichs von 1,7 bis 2,0 Megahertz und/oder von 2,7 bis 3,0 Megahertz sowie geeignet zur Einwirkung auf zumindest einen Teil bei im Behälter (11) befindlicher Waschflüssigkeit enthält.

12. Vorrichtung (3) nach Anspruch 11, wobei sie eine Steuereinheit (41) zur Steuerung der zumindest einen Einrichtung (29, 31) zum Emittieren von elektromagnetischen Wellen beziehungsweise zum Erzeugen von elektromagnetischem Wechselfeld und eine Analyseeinheit (43) zur Analyse der Waschflüssigkeit und/oder des entgasten methanhaltigen Gases beziehungsweise methanhaltigen Gasgemisches enthält.

13. Vorrichtung (3) nach Anspruch 11 oder 12, wobei die Einrichtung (29, 31) zum Emittieren von elektromagnetischen Wellen beziehungsweise zum Erzeugen von elektromagnetischem Wechselfeld Elektroden (29) zur Einwirkung auf zumindest einen Teil bei im Behälter (11) befindlicher Waschflüssigkeit umfasst und das Material der Elektroden (29) ein für Methan katalytisches Material wie insbesondere Nickel enthält.

14. Vorrichtung (3) nach einem der Ansprüche 11 bis 13, wobei sie eine Einrichtung (13; 25) zum Absenken des Drucks auf die Waschflüssigkeit, wie insbesondere eine nachgeschaltete Flashkolonne (13), umfasst.

15. Verwendung einer Vorrichtung (3) nach einem der Ansprüche 11 bis 14 und/oder einer Vorrichtung (3), die einen Behälter (11) mit mindestens einer Einflussöffnung (27) und mindestens einer Ausflussöffnung (33) für Waschflüssigkeit und mindestens eine Einrichtung (29, 31) geeignet zum Emittieren von elektromagnetischen Wellen beziehungsweise zum Erzeugen von elektromagnetischem Wechselfeld innerhalb eines Frequenzbereichs von 0,5 bis 10 Megahertz sowie geeignet zur Einwirkung auf zumindest einen Teil bei im Behälter (11) befindlicher Waschflüssigkeit enthält,
zur Entgasung von Methan aus einer auch Kohlendioxid enthaltenden überwiegend aus Wasser bestehenden Waschflüssigkeit, wie insbesondere bei einer Druckwasserwäsche.

## Claims

1. Process for degassing methane from a scrubbing liquid consisting predominantly of water and also containing carbon dioxide, as in a pressurized water scrubbing in particular, **characterized by**
applying electromagnetic waves or an electromagnetic alternating field to the scrubbing liquid with at least one frequency within a frequency range from 0.5 to 10 megahertz, wherein the binding of the methane in the scrubbing liquid is reducible and/or the movement of the methane in the scrubbing liquid is inducible by the application at the at least one frequency (100).

2. Process according to Claim 1, wherein at least one frequency of the electromagnetic waves or of the electromagnetic alternating field is within a frequency range from 1.7 to 2.0 megahertz or from 2.7 to 3.0 megahertz.

3. Process according to Claim 1 or 2, wherein, on application of the electromagnetic waves or of the electromagnetic alternating field to the scrubbing liquid, a static pressure of more than one bar is acting on the scrubbing liquid.

4. Process according to Claim 3, wherein, on application of the electromagnetic waves or of the electromagnetic alternating field to the scrubbing liquid, a static pressure of two to four bar is acting.

5. Process according to any of Claims 1 to 4, wherein material catalytic to methane, such as nickel in particular, is used in contact with the scrubbing liquid.

6. Process according to any of Claims 1 to 5, having, during or after the process step (100) of applying electromagnetic waves or an electromagnetic alternating field, the process step (110) of lowering the pressure on the scrubbing liquid.

7. Process according to any of Claims 1 to 6, having the further process step (120) of capturing the methane-containing gas or methane-containing gas mixture degassed from the scrubbing liquid.

8. Process according to Claim 7, having the further process step of conducting (130a) the captured methane-containing gas or methane-containing gas mixture onward for another treatment in a pressurized water scrubbing.

9. Process according to any of Claims 1 to 8, which is connected downstream of the absorption step and upstream of the desorption step within a pressurized water scrubbing process for biogas plants.

10. Process according to Claim 9, wherein the process step (110) of lowering the pressure on the scrubbing liquid is present in the last flash column degassing step upstream of the desorption step of the pressurized water scrubbing process.

11. Apparatus (3) for performing a process according to any of Claims 1 to 10 for degassing methane from a scrubbing liquid consisting predominantly of water and also containing carbon dioxide, as in a pressurized water scrubbing in particular, **characterized in that**
- it (3) contains a vessel (11) having at least one inflow opening (27) and having at least one outflow opening (33) for scrubbing liquid,
- it (3) contains at least one device (29, 31) suitable for emitting electromagnetic waves or for generating an electromagnetic alternating field within a frequency range from 1.7 to 2.0 megahertz and/or from 2.7 to 3.0 megahertz, and suitable for contact with at least a portion of the scrubbing liquid present in the vessel (11).

12. Apparatus (3) according to Claim 11, which contains a control unit (41) for controlling the at least one device (29, 31) for emitting electromagnetic waves or for generating an electromagnetic alternating field and an analysis unit (43) for analysis of the scrubbing liquid and/or of the degassed methane-containing gas or methane-containing gas mixture.

13. Apparatus (3) according to Claim 11 or 12, wherein the device (29, 31) for emitting electromagnetic waves or for generating an electromagnetic alternating field comprises electrodes (29) for contact with at least a portion of the scrubbing liquid present in the vessel (11) and the material of the electrodes (29) contains a material catalytic to methane, such as nickel in particular.

14. Apparatus (3) according to any of Claims 11 to 13, which comprises a device (13; 25) for lowering the pressure on the scrubbing liquid, such as a downstream flash column (13) in particular.

15. Use of an apparatus (3) according to any of Claims 11 to 14 and/or of an apparatus (3) containing a vessel (11) having at least one inflow opening (27) and having at least one outflow opening (33) for scrubbing liquid and at least one device (29, 31) suitable for emitting electromagnetic waves or for generating an electromagnetic alternating field within a frequency range from 0.5 to 10 megahertz, and suitable for contact with at least a portion of the scrubbing liquid present in the vessel (11),
for degassing methane from a scrubbing liquid consisting predominantly of water and also containing carbon dioxide, as in a pressurized water scrubbing in particular.

## Revendications

1. Procédé de dégazage de méthane à partir d'un liquide de lavage majoritairement constitué d'eau, contenant également du dioxyde de carbone, tel que notamment lors d'un lavage à l'eau sous pression, **caractérisé par** :
l'action d'ondes électromagnétiques ou d'un champ alternatif électromagnétique sur le liquide de lavage avec au moins une fréquence dans une plage de fréquences allant de 0,5 à 10 mégahertz, la liaison du méthane dans le liquide de lavage pouvant être réduite et/ou le méthane pouvant être mis en mouvement dans le liquide de lavage (100) par l'action à ladite au moins une fréquence.

2. Procédé selon la revendication 1, dans lequel au moins une fréquence des ondes électromagnétique ou du champ alternatif électromagnétique se situe dans une plage de fréquences allant de 1,7 à 2,0 mégahertz ou de 2,7 à 3,0 mégahertz.

3. Procédé selon la revendication 1 ou 2, dans lequel une pression statique supérieure à un bar agit sur le liquide de lavage lors de l'action des ondes électromagnétiques ou du champ alternatif électromagnétique sur le liquide de lavage.

4. Procédé selon la revendication 3, dans lequel une pression statique de deux à quatre bars agit lors de l'action des ondes électromagnétiques ou du champ alternatif électromagnétique sur le liquide de lavage.

5. Procédé selon l'une quelconque des revendications 1 à 4, dans lequel un matériau catalytique pour le méthane, tel que notamment le nickel, est utilisé en contact avec le liquide de lavage.

6. Procédé selon l'une quelconque des revendications 1 à 5, comprenant l'étape de procédé (110) de réduction de la pression sur le liquide de lavage, qui a lieu pendant ou après l'étape de procédé (100) d'action d'ondes électromagnétiques ou d'un champ alternatif électromagnétique.

7. Procédé selon l'une quelconque des revendications 1 à 6, comprenant l'étape de procédé supplémentaire (120) de captage du gaz contenant du méthane ou du mélange gazeux contenant du méthane dégazé du liquide de lavage.

8. Procédé selon la revendication 7, comprenant l'étape de procédé supplémentaire d'acheminement (130a) du gaz contenant du méthane ou du mélange gazeux contenant du méthane capté pour un nouveau traitement dans un lavage à l'eau sous pression.

9. Procédé selon l'une quelconque des revendications 1 à 8, dans lequel il est intercalé après l'étape d'absorption et avant l'étape de désorption dans un procédé de lavage à l'eau sous pression pour des unités de production de biogaz.

10. Procédé selon la revendication 9, dans lequel l'étape de procédé (110) de réduction de la pression sur le liquide de lavage est contenue dans la dernière étape de dégazage en colonne flash avant l'étape de désorption du procédé de lavage à l'eau sous pression.

11. Dispositif (3) pour la réalisation d'un procédé selon l'une quelconque des revendications 1 à 10 pour le dégazage de méthane à partir d'un liquide de lavage majoritairement constitué d'eau, contenant également du dioxyde de carbone, tel que notamment lors d'un lavage à l'eau sous pression, **caractérisé en ce que**
- il (3) contient un contenant (11) comprenant au moins une ouverture d'entrée (27) et au moins une ouverture de sortie (33) pour le liquide de lavage,
- il (3) contient au moins un dispositif (29, 31) approprié pour l'émission d'ondes électromagnétiques ou pour la génération d'un champ alternatif électromagnétique dans une plage de fréquences allant de 1,7 à 2,0 mégahertz et/ou de 2,7 à 3,0 mégahertz, et approprié pour l'action sur au moins une partie du liquide de lavage qui se trouve dans le contenant (11).

12. Dispositif (3) selon la revendication 11, dans lequel il contient une unité de régulation (41) pour la régulation d'au moins un dispositif (29, 31) pour l'émission d'ondes électromagnétiques ou pour la génération d'un champ alternatif électromagnétique, et une unité d'analyse (43) pour l'analyse du liquide de lavage et/ou du gaz contenant du méthane ou du mélange gazeux contenant du méthane dégazé.

13. Dispositif (3) selon la revendication 11 ou 12, dans lequel le dispositif (29, 31) pour l'émission d'ondes électromagnétiques ou pour la génération d'un champ alternatif électromagnétique comprend des électrodes (29) pour l'action sur au moins une partie du liquide de lavage qui se trouve dans le contenant (11), et le matériau des électrodes (29) contient un matériau catalytique pour le méthane, tel que notamment le nickel.

14. Dispositif (3) selon l'une quelconque des revendications 11 à 13, dans lequel il comprend un dispositif (13, 25) pour la réduction de la pression sur le liquide de lavage, tel que notamment une colonne flash en aval (13).

15. Utilisation d'un dispositif (3) selon l'une quelconque des revendications 11 à 14 et/ou d'un dispositif (3) qui comprend un contenant (11) comprenant au moins une ouverture d'entrée (27) et au moins une ouverture de sortie (33) pour le liquide de lavage et au moins un dispositif (29, 31) approprié pour l'émission d'ondes électromagnétiques ou pour la génération d'un champ alternatif électromagnétique dans une plage de fréquences allant de 0,5 à 10 mégahertz, et approprié pour l'action sur au moins une partie du liquide de lavage qui se trouve dans le contenant (1), pour le dégazage de méthane à partir d'un liquide de lavage majoritairement constitué d'eau, contenant également du dioxyde de carbone, tel que notamment lors d'un lavage à l'eau sous pression.
